# EUROPÄISCHE PATENTSCHRIFT

(11) **EP 1 922 096 B1**
(45) Veröffentlichungstag und Bekanntmachung des Hinweises auf die Patenterteilung: **24.09.2014**
(21) Anmeldenummer: 06776374.8
(22) Anmeldetag: 24.07.2006
(51) Int. Cl.: A61M 1/02

(54) **VORRICHTUNG ZUR FILTRATIONSÜBERWACHUNG VON BLUTKONSERVEN**
DEVICE FOR MONITORING THE FILTRATION OF UNITS OF STORED BLOOD
DISPOSITIF POUR SURVEILLER LA FILTRATION DE SANG CONSERVE

(30) Priorität: 26.08.2005 DE 102005040518
(43) Veröffentlichungstag der Anmeldung: 21.05.2008
(73) Patentinhaber: Fraunhofer-Gesellschaft zur Förderung der angewandten Forschung e.V., 80686 München (DE)
(72) Erfinder: BIESINGER, Bernd, 70437 Stuttgart (DE); SUGG, Udo, 70184 Stuttgart (DE); BAUMGARTNER, Werner, 74391 Erligheim (DE)
(74) Vertreter: Rösler, Uwe
(86) Internationale Anmeldenummer: PCT/EP2006/007262
(87) Internationale Veröffentlichungsnummer: WO 2007/022843

(56) Entgegenhaltungen:
- EP-A2- 1 048 305
- DE-C1- 10 104 686
- US-A1- 2001 054 356
- US-A1- 2003 150 508

## Beschreibung

### Technisches Gebiet

Die Erfindung bezieht sich auf eine Vorrichtung sowie ein Verfahren zur Filtrationsüberwachung von Blutkonserven mit einer Befestigungsvorrichtung, an der ein Beutelfiltersystem anbringbar ist, das ein mit einer Blutkonserve gefülltes Behältnis aufweist, an dem in reihenhafter, vertikaler Abfolge eine über eine erste Zuleitung mit dem Behältnis verbundene Filtereinheit sowie ein über eine zweite Zuleitung mit der Filtereinheit verbundenes Sammelbehältnis vorgesehen sind.

### Stand der Technik

Seit dem Jahr 2001 ist in Deutschland die Filtration von Blutspenden, entweder als Vollblutspende oder als Konzentrat von roten Blutkörperchen, den sogenannten Erythrozytenkonzentraten, vorgeschrieben. Bei dieser so genannten Leukozytendepletion wird mittels eines Adhäsionsfilters die Anzahl der in den Konzentraten noch verbliebenen weißen Blutkörperchen (Leukozyten) von typischerweise 10⁹ auf unter 10⁶ bei einem mittleren Volumen von 250ml reduziert. Dies hat zum Ziel die pharmazeutische Qualität und die Verträglichkeit dieser Blutprodukte weiter zu erhöhen. Leukozyten stehen außerdem im Verdacht, an der Übertragung der neuen Variante der Creutzfeldt-Jakob-Krankheit (nvCJK) beteiligt zu sein, so dass durch eine deutliche Reduzierung des Leukozytenanteils in Blutkonzentraten im Wege der Filtration eine entsprechende Übertragung vermieden oder zumindest stark eingeschränkt werden könnte.

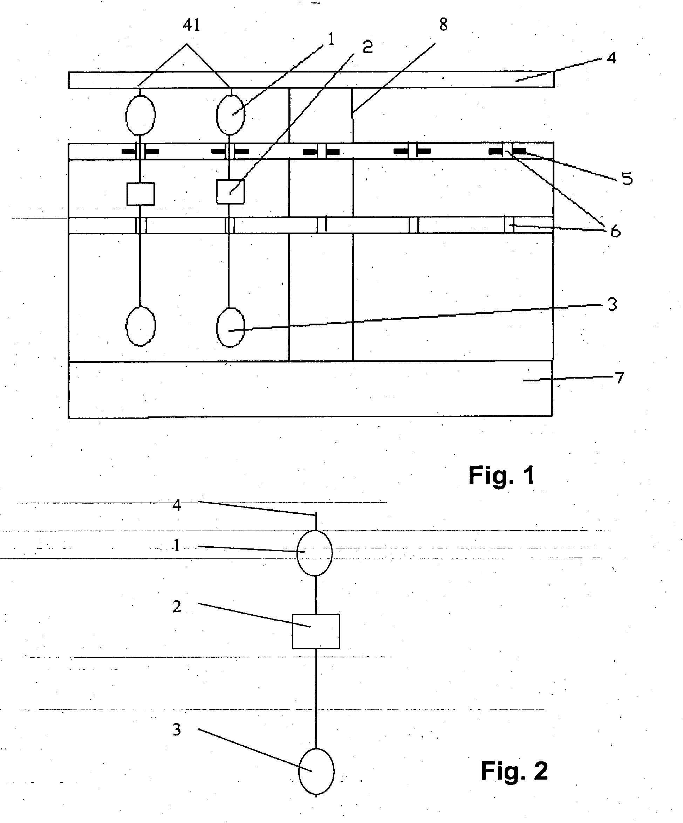

Die Filtrationen erfolgen in geschlossenen und sterilen Beutelfiltrationssystemen, so genannten in-line Filtrationssystemen. Ein derartiges Beutelfiltrationssystem ist in Figur 2 schematisiert dargestellt. Ein Beutel mit Vollblut oder Erythrozytenkonzentrat 1 ist hierbei über einen Schlauch und einem zwischengeschalteten Adhäsionsfilter 2 mit einem weiteren leeren Beutel 3, den so genannten Sammelbehältnis verbunden. Der Filtrationsvorgang erfolgt durch Aufhängen des mit Blut gefüllten Beutels 1 an einer Befestigungsvorrichtung 4, wobei der Adhäsionsfilter 2 und das daran angeschlossene Sammelbehältnis 3 schwerkraftbedingt unterhalb der Befestigungsvorrichtung 4 frei hängen. Das Blut läuft durch die Schwerkraft getrieben aus dem oberen Beutel 1 aus und durchströmt den Filter 2, wobei das gefilterte Blut bzw. das gefilterte Erythrozytenkonzentrat in das Sammelbehältnis 3 fließt.

Die Filtrierzeit ist hierbei ein die Qualität entscheidendes Kriterium. Bei verlängerter Filtrationsdauer kann auf einen erhöhten Anteil an Koagulationsprodukten geschlossen werden; bei verringerter Dauer auf eine Fehlfunktion des Filters.

Die Überwachung und Dokumentation der Filtration von Blutpräparaten erfolgt derzeit hauptsächlich manuell, d.h. in Gegenwart einer Person. Das ausführende Personal stoppt die Zeit der Filtration hierbei von Hand. Bei der Vielzahl der zu verarbeitenden Blutspenden und aufgrund paralleler Tätigkeiten des durchführenden Personals ist die jedoch die Überwachung und Dokumentation jedes einzelnen Filtrationsvorganges nicht immer gewährleistet.

Derzeit ist lediglich eine einzige Anlage bekannt, die die Filtrationsdauer technisch zu erfassen vermag. Hierbei handelt es sich um ein System, wie es bspw. aus der DE 103 15 484 A1 hervorgeht, das über Wägezellen die Gewichtsreduktion der Blutkonserve in dem oberen Beutel misst. Nachteilig ist bei dieser Ausführung, dass eine Gewichtsabnahme der Blutkonserve nur dann gemessen werden kann, wenn der untere Beutel des Filtrationssystems, das Sammelbehältnis, auf einer Unterlage abgelegt wird. Einige Hersteller von Filtrationssystemen sind in ihren Vorschriften jedoch nicht eindeutig, ob dies zulässig ist. Für eine sichere Filtration sollten die Blutfiltrationssysteme frei hängen.

Ein weiterer Nachteil sowohl bei der manuellen Überwachung als auch bei der technischen Variante mit Wägezellen ist, dass die Filter der Systeme ungeschützt gegenüber äußeren mechanischen Erschütterungen sind. Die Filter sind als Adhäsionsfilter ausgeführt, die nur dann einwandfrei funktionieren, wenn sie nicht erschüttert oder bewegt werden.

Zudem variieren die Beutelfiltrationssysteme je nach Hersteller in ihrer Gesamtlänge und in ihrem Abstand Beutel zu Filter. Es gibt Systeme mit einer Gesamtlänge von über 2 Metern. Diese Systeme können nur an Anlagen eingesetzt werden, die in ihrer Aufhängungshöhe veränderbar ausgelegt sind.

In Ergänzung geht aus der DE 198 17 328 C2 eine Vorrichtung sowie ein Verfahren zur Herstellung eines leukozyten- und erythrozytenarmen Thrombozytenpräparates hervor. Die Vorrichtung umfasst eine Blutbeutelpresse, die einen Blutbeutel zwischen einer stehenden Platte und einer durch eine Kolben-Zylindereinheit angetriebene bewegliche Platte abpresst, eine erste Zuleitung, die den Blutbeutel mit einem Leukozytendepletionsfilter verbindet, eine zweite Zuleitung, die den Leukozytendepletionsfilter mit einem Sammelbeutel verbindet, einen an der ersten Zuleitung angebrachten Erythrozytensensor, sowie eine Steuereinheit, durch welche eine Schweißeinheit und die Kolben-Zylindereinheit angesteuert wird. Der Erythrozytensensor, der auch als Rot- oder Hb-Detektor bezeichnet wird, erfasst die in der durch die Zuleitung fließenden Lösung enthaltene Erythrozytenkonzentration. Sobald eine vorgegebene Erythrozytenkonzentration überschritten wird, stoppt die Steuereinheit den Abpressvorgang durch einen entsprechenden Befehl an die Kolben-Zylindereinheit und betätigt daraufhin die Schweißeinheit derart, dass die Zuleitung vor dem Leukozytendepletionsfilter abgetrennt und verschweißt wird.

Schließlich ist der DE 196 38 104 C2 eine Vorrichtung sowie ein Verfahren zur Herstellung von Leukozyten-abgreicherten Produkten aus Leukozyten enthaltender Flüssigkeit zu entnehmen.

Aus US 2003/0150508 A1 ist ein System zum automatischen Handling von Beutelsystemen bekannt, bei dem die Beutelsysteme aus einem Zustand, in dem keine Filtration stattfindet, automatisch in einen Zustand überführt werden, in dem die Filtration stattfindet. Die beiden Enden jedes Beutelsystems sind an den Achsen 29 bzw. 30 zuordenbaren Befestigungsmitteln befestigt.

### Darstellung der Erfindung

Der Erfindung liegt die Aufgabe zugrunde eine Vorrichtung sowie ein Verfahren zur Filtrationsüberwachung von Blutkonserven mit einer Befestigungsvorrichtung, an der ein Beutelfiltersystem anbringbar ist, das ein mit einer Blutkonserve gefülltes Behältnis aufweist, an dem in reihenhafter, vertikaler Abfolge eine über eine erste Zuleitung mit dem Behältnis verbundene Filtereinheit sowie ein über eine zweite Zuleitung mit der Filtereinheit verbundenes Sammelbehältnis vorgesehen sind, und mit einem Sensorsystem im Bereich der ersten und/oder zweiten Zuleitung, das einen Blutdurchfluss durch die Zuleitung detektiert, derart weiterzubilden, dass eine Zeitmessung des vollständigen Blutdurchflusses durch den Filter exakt erfassbar ist, ohne die Notwenigkeit eines personellen Einsatzes. Die hierfür zu treffenden Maßnahmen sollen es überdies erlauben Blutfiltrationssysteme von unterschiedlichen Herstellern und unterschiedlichen Längen zu überwachen und vor äußeren mechanischen Einflüssen zu schützen. Ferner soll es möglich sein eine Vielzahl von zeitgleich ablaufenden Blutfiltrationen zu erfassen und zu überwachen.

Die Lösung der Erfindung ist im Anspruch 1 angegeben. Den Erfindungsgedanken vorteilhaft weiterbildende Merkmale sind in den Unteransprüchen sowie in der nachfolgenden Beschreibung angegeben.

Lösungsgemäß zeichnet sich eine Vorrichtung zur Filtrationsüberwachung von Blutkonserven mit einer Befestigungsvorrichtung, an der ein Beutelfiltersystem anbringbar ist, das ein mit einer Blutkonserve gefülltes Behältnis aufweist, an dem in reihenhafter, vertikaler Abfolge eine über eine erste Zuleitung mit dem Behältnis verbundene Filtereinheit sowie ein über eine zweite Zuleitung mit der Filtereinheit verbundenes Sammelbehältnis vorgesehen sind, und mit einem Sensorsystem im Bereich der ersten und/oder zweiten Zuleitung, das einen Blutdurchfluss durch die Zuleitung detektiert, dadurch aus, dass eine Auswerteinheit vorgesehen ist, die mittel- oder unmittelbar mit dem Sensorsystem verbunden ist und eine Zeitmessung zur Bestimmung der Zeitdauer einer vollständigen Blutfiltration durchführt, d.h. die zeitliche Dauer, die die in dem Behältnis bevorratete Blutkonserve benötigt durch die Filtereinheit in das Sammelbehältnis zu gelangen, und dass eine Speichereinheit vorgesehen ist, die die Zeitdauer für die vollständige Blutfiltration abspeichert. Die Vorrichtung zeichnet sich weiter dadurch aus, dass im Bereich der ersten und/oder zweiten Zuleitung Führungsmittel vorgesehen sind, in die die Zuleitungen längsverschieblich klemmbar sind, und
dass der Sammelbehälter derart über die zweite und erste Zuleitung sowie die zwischengeschaltete Filtereinheit an der Befestigungsvorrichtung angeordnet ist, dass der Sammelbehälter ausschließlich an der Zuleitung frei schwebend hängt

Durch die sensorielle Überwachung des anhaltenden Blutdurchflusses, die mittels optischer, kapazitiver oder schalltechnischer Sensoren vorgenommen werden kann, ist es möglich die Sensorsignale einer Speicher- und Auswerteeinheit zuzuführen, in der die Signale aufbereitet und zum Zwecke der Bestimmung der zeitlichen Dauer des Blutdurchflusses ausgewertete werden können. Durch den Einsatz rechnergestützter Auswerteinheiten, wie bspw. PC-Rechner, eröffnet sich die Möglichkeit, Blutfiltrationen bei einer Vielzahl vorgesehener und mit entsprechender Sensorik ausgerüsteter Blutfiltrationssystemen simultan durchzuführen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Schematisierte Darstellung einer lösungsgemäßen Vorrichtung,
- Fig. 2: schematisierte Darstellung einer an sich bekannten Vorrichtung gemäß Stand der Technik.

### Wege zur Ausführung der Erfindung, gewerbliche Verwendbarkeit

Figur 1 zeigt schematisiert einer Vorrichtung zur Überwachung der Blutfiltration. An einer Befestigungsvorrichtung 4, die höhenverstellbar ausgelegt ist, ist eine Vielzahl von Befestigungsmöglichkeiten 41 vorgesehen, an die jeweils freitragend ein Blutfiltrationssystem, wie es vorstehend unter Bezugnahme auf Figur 2 beschrieben ist, lösbar fest anbringbar ist.

Die Detektion des Blutflusses und somit die Aufnahme der Filtrationsdauer erfolgt über eine optische Sensorik in Form einer Durchlichtschranke 5 an einem durchsichtigen Verbindungsschlauch oberhalb des Filters 2. Eine Messung startet sofort nach der ersten Detektion des Blutflusses. Alternativ oder in Kombination hierzu kann die Detektion auch über einen kapazitiven Sensor 5 oder einen Ultraschallsensor (nicht dargestellt) erfolgen, insbesondere dann, wenn die Verbindungsschläuche nicht durchsichtig ausgeführt sind.

Die Auswerteelektronik für die Sensoren 5 ist innerhalb eines Installationsschrankes 7 über ein Bussystem mit einem Industrie-PC verbunden, der die Steuerung der Gesamtanlage übernimmt. Mit dem Installationsschrank 7 ist über einen Vertikalträger 8 die Befestigungsvorrichtung 4 verbunden, so dass der Installationsschrank 7 und die Befestigungsvorrichtung 4 als eine Einheit ausgeführt ist, die bspw. auf Rollen gelagert an beliebige Orte verschoben werden kann.

Neben einer Visualisierung der einzelnen Filtrierstationen durch den PC-Rechner hat der Anwender darüber hinaus die Möglichkeit spezifische Einstellungen vorzunehmen, wie die Vorgabe von minimal und maximal zulässiger Filtrationsdauer sowie die Zuordnung dieser Vorgaben zu den jeweiligen Filtrationssystemen . Der Steuerrechner vergleicht die gemessene Filtrationsdauer mit den hinterlegten Werten und bestimmt so den Status der einzelnen Filtrationen.

Da die Beutelfiltrationssysteme je nach Hersteller in ihrer Gesamtlänge und in ihrem Abstand Beutel 1 zu Filter 2 variieren ist das System mit einer elektrisch in der Höhe verstellbaren Beutelaufhängungsvorrichtung 4 ausgerüstet, die sich universell an die verschiedenen Filtrationssysteme anpassen lässt. Dies ist insbesondere bei solchen Systemen von Bedeutung, bei denen der untere Beutel nach Herstellervorschrift frei hängen muss und nicht abgelegt werden sollte.

Ober- und unterhalb des Filters sind die Verbindungsschläuche zu den Beuteln in dafür vorgesehene Nuten 6 exakt geführt. Die Breite der Nuten entspricht dem Durchmesser der Schläuche des Filtrationssystems, so dass die Schläuche leicht eingeklemmt werden. Dies fixiert den Filter 2 in seiner Position, was insbesondere deshalb von Bedeutung ist, da die Funktion eines Adhäsionsfilters 2 nur dann gewährleistet ist, wenn dieser während des Filtrationsvorganges nicht erschüttert oder bewegt wird.

Die Bedienung des Systems erfolgt über Bedienelemente mit Leuchtmitteln zur Statusanzeige an den einzelnen Filtrierstationen, sowie über Eingriffsmöglichkeiten über den PC-Rechner. Die eindeutige Identifikation der einzelnen Blutspenden erfolgt über eine integrierte Barcode- Erfassung mittels eines Funkbarcodescanners. Es werden sämtliche Filtrationszeiten mit Barcodekennung, Anfangszeit, Endzeit, Dauer, Bedienerkurzzeichen, Typ des Filtrationssystems, Zuordnung zur Filtrationsstation sowie der abschließende Filtrationsstatus in einer Datenbank dokumentiert. Diese Informationen können über eine Netzwerkanbindung an lokale Qualitätssicherungs- und Dokumentationssysteme exportiert werden.

Die Anpassung der Anlage an verschiedene Filtrationssysteme kann der Anwender über individuell konfigurierbare Einstellungen vornehmen und dauerhaft hinterlegen. So können für jedes Filtrationssystem die Empfindlichkeit der Sensorik, die minimal und maximal zulässige Filtrationsdauer nach Herstellervorschrift sowie verschiedene Handlingzeiten hinterlegt und bei Bedarf eingelesen werden.

### Bezugszeichenliste

- 1: Beutel
- 2: Filtereinheit, Adhäsionsfilter
- 3: Beutel, Sammelbehälter
- 4: Befestigungsvorrichtung
- 41: Befestigungsmöglichkeit
- 5: Sensor
- 6: Nut, Führung
- 7: Installationsschrank
- 8: Vertikalträger

## Patentansprüche

1. Vorrichtung zur Filtrationsüberwachung von Blutkonserven mit einer Befestigungsvorrichtung, an der ein Beutelfiltersystem anbringbar ist, das ein mit einer Blutkonserve gefülltes Behältnis (1) aufweist, an dem in reihenhafter, vertikaler. Abfolge eine über eine erste Zuleitung mit dem Behältnis verbundene Filtereinheit (2) sowie ein über eine zweite Zuleitung mit der Filtereinheit verbundenes Sammelbehältnis (3) vorgesehen sind, und mit einem Sensorsystem (5) im Bereich der ersten und/oder zweiten Zuleitung, das einen Blutdurchfluss durch die Zuleitung detektiert,
**dadurch gekennzeichnet, dass** eine Auswerteinheit vorgesehen ist, die mittel- oder unmittelbar mit dem Sensorsystem verbunden ist und eine Zeitmessung zur Bestimmung der Zeitdauer einer vollständigen Blutfiltration durchführt, d.h. die zeitliche Dauer, die die in dem Behältnis bevorratete Blutkonserve benötigt durch die Filtereinheit in das Sammelbehältnis zu gelangen,
dass eine Speichereinheit vorgesehen ist, die die Zeitdauer für die vollständige Blutfiltration abspeichert,
dass im Bereich der ersten und/oder zweiten Zuleitung Führungsmittel (6) vorgesehen sind, in die die Zuleitungen längsverschieblich klemmbar sind, und
dass der Sammelbehälter derart über die zweite und erste Zuleitung sowie die zwischengeschaltete Filtereinheit an der Befestigungsvorrichtung angeordnet ist, dass der Sammelbehälter ausschließlich an der Zuleitung frei schwebend hängt.

2. Vorrichtung nach Anspruch 1,
**dadurch gekennzeichnet, dass** das Sensorsystem im Falle einer aus transparenten Material bestehenden Zuleitung einen optischen Sensor aufweist.

3. Vorrichtung nach Anspruch 1 ,
**dadurch gekennzeichnet, dass** das Sensorsystem einen kapazitiven Sensor aufweist, der einen Blutdurchfluss durch die Zuleitung im Wege sich ändernder dielektrischer Verhältnisse erfasst.

4. Vorrichtung nach Anspruch 1,
**dadurch gekennzeichnet, dass** das Sensorsystem einen Ultraschallsensor aufweist, der durch Ultraschalleinkopplung in die Zuleitung den Blutdurchfluss im Wege sich ändernde Schalllaufzeiten erfasst.

5. Vorrichtung nach einem der Ansprüche 1 bis 4,
**dadurch gekennzeichnet, dass** die Speichereinheit Teil einer Rechnereinheit, vorzugsweise ein PC-Rechner ist.

6. Vorrichtung nach einem der Ansprüche 1 bis 6,
**dadurch gekennzeichnet, dass** die Befestigungsvorrichtung in der vertikalen Lage verstellbar ist.

7. Vorrichtung nach einem der Ansprüche 1 bis 6,
**dadurch gekennzeichnet, dass** die Befestigungsvorrichtung Teil eines Rahmengestells ist, das eine Vielzahl einzelner Befestigungsvorrichtungen nebeneinander geordnet aufweist,
dass eine Rechnereinheit vorgesehen ist, an der sämtliche an den jeweiligen Befestigungsvorrichtungen angebrachten Beutelfiltrationssysteme gewonnenen Zeitdauern erfassbar und abfragbar sind.

## Claims

1. Device for monitoring the filtration of units of stored blood, having a fixing device to which a pouch filter system can be applied, said system comprising a container (1) filled with a unit of stored blood and provided with, in a serial vertical sequence, a filter unit (2) connected to the container by means of a first supply line, and a collecting container (3) connected to the filter unit by means of a second supply line, and having a sensor system (5) in the region of the first and/or second supply line which detects a flow of blood through the supply line,
**characterized in that**
an evaluation unit is directly or indirectly connected to the sensor system, and is used to carry out a time measurement in order to determine the duration of a complete blood filtration, i.e. the period of time required by the unit of blood stored in the container to reach the collecting container via the filter unit,
that a memory unit is provided for storing the duration of the entire blood filtration,
that in the region of the first and/or second supply line (6), guiding means are provided into which the supply lines can be clamped such that they are longitudinally displaceable, and
that the collecting container is arranged across the second and first supply line and the intermediate filter unit is arranged on the mounting device in such a manner that the collecting container is suspended from the supply pipe alone in a free-floating manner.

2. The device according to claim 1, **characterized in that** in the case of a supply line composed of transparent material, the sensor system has an optical sensor.

3. The device according to claim 1, **characterized in that** the sensor system comprises a capacitive sensor which detects a flow of blood through the supply line by the method of varying dielectric conditions.

4. The device according to claim 1, **characterized in that** the sensor system comprises an ultrasonic sensor which detects the flow of blood by ultrasonic coupling into the supply line by the method of varying sound transit times.

5. The device according to any one of claims 1 to 4, **characterized in that** the memory unit is part of a computer unit, preferably a PC.

6. The device according to any one of claims 1 to 5, **characterized in that** the mounting device is adjustable in the vertical position.

7. The device according to any one of claims 1 to 6,
**characterized in that**
the mounting device is part of a chassis frame, which comprises a plurality of individual mounting devices arranged next to one another,
that a computer unit is provided, in which all of the durations obtained from pouch filtration systems mounted on mounting devices can be detected and queried.

## Revendications

1. Dispositif destiné au contrôle de filtration de conserves de sang avec un dispositif de fixation sur lequel un système de filtre à poche peut être placé qui comporte un récipient (1) rempli d'une conserve de sang sur lequel sont prévus une unité de filtre (2) raccordée au récipient par un premier conduit d'alimentation ainsi qu'un récipient de collecte (3) raccordé à l'unité de filtre par un deuxième conduit d'alimentation, alignés en succession verticale et avec un système de détection (5) dans la zone du premier et/ou deuxième conduit d'alimentation qui détecte le un débit de sang à travers le conduit d'alimentation
**caractérisé en ce qu'**une unité d'évaluation est prévue qui est raccordée directement ou indirectement au système de détection et effectue une mesure de temps pour déterminer la durée d'une filtration de sang complète, c'est-à-dire la durée temporelle que nécessite la conserve de sang approvisionnée dans le récipient pour parvenir par l'unité de filtre dans le récipient de collecte,
**en ce qu'** une unité de mémorisation est prévue qui mémorise la durée de temps pour la filtration du sang complète,
**en ce que** dans la zone du premeir et/ou deuxième conduit d'alimentation des moyens de guidage (6) sont prévus dans lesquels les conduits d'alimentation peuvent être serrés de façon déplaçable longitudinalement, et
**en ce que** le récipient de collecte sur le deuxième et premier conduit d'alimentation ainsi que l'unité de filtre intercalée sur le dispositif de fixation de sont disposés de telle sorte que le récipient de collecte est librement suspendu exclusivement au conduit d'alimentation.

2. Dispositif selon la revendication 1 **caractérisé en ce que** le système de détection comporte un capteur optique dans le cas d'un conduit d'alimentation composé d'un matériau transparent.

3. Dispositif selon la revendication 1 **caractérisé en ce que** le système de détection comporte un capteur capacitif qui saisit un débit de sang à travers le conduit d'alimentation sous la forme de rapports diélectriques variables.

4. Dispositif selon la revendication 1 **caractérisé en ce que** le système de capteur comporte un capteur à ultrasons qui saisit par injection d'ultrasons dans le conduit d'alimentation le débit de sang sous la forme de temps de parcours du son variables.

5. Dispositif selon une quelconque des revendications 1 à 4 **caractérisé en ce que** l'unité de mémorisation est une partie d'une unité de calcul, de préférence un calculateur d'ordinateur privé.

6. Dispositif selon une quelconque des revendications 1 à 6 **caractérisé en ce que** le dispositif de fixation est réglable dans la position verticale.

7. Dispositif selon une quelconque des revendications 1 à 6
**caractérisé en ce que** le dispositif de fixation est une partie d'un châssis qui comporte une pluralité de dispositifs de fixation individuels disposés les uns près des autres,
**en ce qu'**une unité de calcul est prévue sur laquelle il est possible de saisir et consulter toutes les durées de temps acquises sur les systèmes de filtre à poche disposés sur les dispositifs de fixation respectifs.
